# EUROPEAN PATENT SPECIFICATION

(11) **EP 4 140 490 B1**
(45) Date of publication and mention of the grant of the patent: **07.01.2026**
(21) Application number: 21866938.0
(22) Date of filing: 29.04.2021
(51) Int. Cl.: A61K 31/7048, A61P 17/14, A61K 8/60, A61Q 7/00, A61Q 5/00

(54) **COMPOSITION FOR AMELIORATION, PREVENTION OR TREATMENT OF HAIR LOSS, COMPRISING CATECHIN 7-O-BETA-D-APIOFURANOSIDE AS ACTIVE INGREDIENT**
ZUSAMMENSETZUNG ZUR LINDERUNG, VORBEUGUNG ODER BEHANDLUNG VON HAARAUSFALL MIT CATECHIN 7-O-BETA-D-APIOFURANOSID ALS WIRKSTOFF
COMPOSITION POUR L'AMÉLIORATION, LA PRÉVENTION OU LE TRAITEMENT DE LA PERTE DE CHEVEUX, COMPRENANT LA CATÉCHINE 7-O-BETA-D-APIOFURANOSIDE COMME INGRÉDIENT ACTIF

(30) Priority: 08.09.2020 KR 20200114893; 08.09.2020 KR 20200114895
(43) Date of publication of application: 01.03.2023
(73) Proprietor: Anpoly Inc., Gyeongsangbuk-do 37673 (KR)
(72) Inventor: CHOI, Sun Eun, Gwangju 62322 (KR)
(74) Representative: Gill Jennings & Every LLP
(86) International application number: PCT/KR2021/005465
(87) International publication number: WO 2022/055067

(56) References cited:
- JP-A- 2000 095 649
- KR-A- 20060 095 062
- JUNG, MEE-JUNG ET AL.: "Reducing Power and α-Glucosidase Inhibitory profiles of (-)-Catechin and Its glycoside", NATURAL PRODUCT SCIENCES, vol. 38, no. 4, 2007, pages 358 - 362, XP053014039
- JUNG, M.J. ; HEO, S.I. ; WANG, M.H.: "HPLC analysis and antioxidant activity of Ulmus davidiana and some flavonoids", FOOD CHEMISTRY, ELSEVIER LTD., NL, vol. 120, no. 1, 1 May 2010 (2010-05-01), NL , pages 313 - 318, XP026799358, ISSN: 0308-8146
- PARK YONG JOO, KIM DONG MIN, JEONG MI HO, YU JAE SIK, SO HAE MIN, BANG IN JAE, KIM HA RYONG, KWON SEUNG-HWAN, KIM KI HYUN, CHUNG K: "(–)-Catechin-7-O-β-d-Apiofuranoside Inhibits Hepatic Stellate Cell Activation by Suppressing the STAT3 Signaling Pathway", CELLS, vol. 9, no. 1, 20 December 2019 (2019-12-20), pages 30, XP055910567, DOI: 10.3390/cells9010030

## Description

### Technical Field

The present invention relates to a composition for use in preventing or treating alopecia, in which the composition includes catechin 7-O-β-D-apiofuranoside as an active ingredient.

### Background Art

Although hair loss does not have a pathological problem compared to other pathological diseases, it affects the appearance and has a great impact on people who suffer from hair loss as a social and psychological problem. In the past, hair loss was recognized as an aging phenomenon, but recently it has been caused by various factors such as genetic factors, stress, nutritional imbalance, changes in social activities, diseases, childbirth, changes in eating habits, irregular life, and excessive use of chemical hairdressing agents. Types of hair loss are largely classified into male pattern hair loss, female pattern hair loss, alopecia areata, postpartum hair loss, and hair loss due to seborrheic scalpitis, and the type of hair loss with the highest rate among them is male pattern hair loss.

Male pattern hair loss is caused by genetic causes, and when hair loss starts early, the degree of hair loss tends to become more severe. In addition to genetic causes, testosterone, a male hormone, is changed to dihydrotestosterone (DHT) by 5α-reductase, and DHT, which is produced, shrinks dermal papilla cells and slows down cell division, resulting in thinning hair or hair loss.

Hair loss caused by stress appears in the form of alopecia areata. Alopecia areata is a disease in which hair is lost from some or all parts of the body. Commonly, bald spots of the size of a few coins appear on the scalp, which may be caused by psychological stress. In some cases, hair on the scalp or hair on the whole body such as beard or eyebrows disappears, resulting in permanent hair loss.

In addition, hair loss due to stress may appear in the form of telogen hair loss, and telogen hair loss is hair loss that occurs when the normal growth cycle of hair enters telogen phase. Human hair repeats growth and extinction every 3 to 6 years. Hair that has grown for 3 to 6 years degenerates over about 3 to 4 weeks, and then enters the telogen phase in which hair loss lasts for about 3 to 5 months. Normally, about 10% (about 10,000 strands) of hair enters the telogen phase, and hair loss in the form of hair falling out as the hair enters the telogen phase in this form is called telogen hair loss.

IGF-1 promotes the proliferation of epithelial cells in culture, and increases the length of hair follicle tissue, thereby acting as a factor that promotes hair growth, and is an important growth factor involved in the regulation of hair growth (Itami et al., 1995; Hibino & Nishiyama, 2004).

IGF-1 growth factor is reported to be involved in the action of testosterone on hair due to its increased expression by male hormones, and to play an important role in the mechanism of action that causes androgenetic alopecia. This factor helps to prevent aging, ameliorate dementia and depression, prevent lifestyle diseases such as diabetes or high blood pressure, increase immunity and bone density, and prevent skin aging. This factor is used to treat hair loss by inducing conversion from a telogen phase to an anagen phase of hair.

When TGF-β1, which induces catagen development, was injected into the skin of the back of the mouse, hair follicles proliferated and the number of keratinocytes decreased, and the number of TUNEL cells increased in TGF-β1-treated mice compared to the control group. In addition, it was found that dual visualization of TGF-β type II receptor (TGFRIl) and TUNEL reactivity showed colocalization of apoptotic nucleus and TGFRII in catagen hair follicles. Accordingly, it has been suggested that TGF-βRII agonists and antagonists may be provided as useful treatments for human hair growth disorders based on early or delayed catagen hair loss and hirsutism (Foitzik K, Lindner G, Mueller-Roever S, Maurer M, Botchkareva N, Botchkarev V, Handjiski B, Metz M, Hibino T, Soma T, Dotto GP, Paus R(2000). Control of murine hair follicle regression (catagen) by TGF-beta1 in vivo. FASEB journal, 14(5): 752-812).

Shin et al. (2013) reported that androgenetic alopecia is closely related to TGF-β1 secretion (Shin H, Yoo HG, Inui S, Itami S, Kim IG, Cho AR, Lee DH, Park WS, Kwon O, Cho KH, Won CH(2013). Induction of transforming growth factor-beta 1 by androgen is mediated by reactive oxygen species in hair follicle dermal papilla cells. BMB Reports, 46(9): 460-464.).

Androgen receptor-transformed rat bladder papilla cells (DP-6) showed increased ROS production after androgen treatment, and TGF-β1 secretion was increased by androgen treatment in DP-6, while androgen-induced TGF-β1 was significantly inhibited by N-acetylcysteine. It has been reported that the induction of TGF-β1 by androgen is mediated by ROS of hair follicle DPCs, suggesting the use of antioxidant therapy for the treatment of androgenetic alopecia.

Regarding the effect of growth factors on hair growth, growth factors such as epidermal growth factor (EGF), insulin-like growth factor (IGF), fibroblast growth factor (FGF), and vascular endothelial growth factor (VEGF) have presently been found to be involved in hair growth by acting on specific sites in hair follicles through various experiments (Tsuboi R(1997). Growth factors and hair growth. Kor J Invest Dermatil, 4(2): 103-108). In 1994, Hervert's research team reported that mice lacking the fibroblast growth factor (FGF)-5 gene had an abnormally long anagen phase and relatively long hair, and FGF-7, IGF-1, and HGF are paracrine growth factors that promote hair growth (paracrine growth factor) (Hervert JM, Rosenquist T, Gotz J, Martin GR(1994). FGF-5 as a regulator of the hair growth cycle: Evidence from targeted and spontaneous mutation. Cell, 78: 1017-1025).

Jiang et al. (1995) found that among the endocrine factors that affect hair growth and hair loss, TGF-β1 interferes with hair growth in the anagen stage and causes hair to enter the catagen stage earlier than normal, thus leading to hair loss, and that growth factors such as IGF-1, TGF-β2, EGF, and FGF promote hair growth and prevent hair loss by preventing degeneration of hair cells (Jiang H, Yamamoto S, Kato R(1995). Induction of anagen in telogen mouse skin by topical application of FK506, a potent immunosuppressant. J. Invest. Dermatol, 104: 523-525).

In a study by Hyun-Wook Na and others (2006), it was reported that hair growth has a closer relationship with blood circulation, and insulin, a kind of hormone, and IGF-1 and EGF, which are growth factors, promote hair growth and affect hair follicle growth (Hyun-Wook Na, Chang-Hyun Lee, Jin Kwon, Moon-Won Lee, Han-Sol Jung, and Gwang-Gyu Lee (2006). Journal of Physiology & Pathology in Korean Medicine, 20(2), 428-435).

Minoxidil was first developed as a vasodilator to treat hypertension in the early 1970s, but was developed as a hair growth promoter since it was reported to cause hirsutism as a side effect. The mechanism for hair-restoring effects was not clearly established, but an increase in supply of nutriments due to vasodilatation has been reported to induce hair growth. However, minoxidil is not effective on the front of the scalp that has already become bald, and it may not be effective unless steadily applied to the scalp twice a day for 6 to 12 months. As side effects of minoxidil, edema, tachycardia, local exfoliation, dermatitis, soreness at the site of administration, erythema, skin peeling, itching, dryness, skin exfoliation, contact dermatitis, etc. have been reported. Also, it has been reported that hirsutism may be induced. In addition, there may be a phenomenon in which hair loss becomes more severe due to an increase in telogen hair loss (shedding) as minoxidil affects hair cycle. Also, there is a shortcoming in that the period of returning to the state before treatment is short when medication is stopped.

In Jung et al., 'Reducing Power and α-Glucosidase Inhibitory profiles of (-)-Catechin and Its glycoside', Natural product sciences (Kor. J. Pharmacogn.), vol. 38, no. 4, 2007, pages 358-362, (-)-catechin, (-)-catechin-7-O-β-D-apiofuranoside, and (-)-catechin-7-O-β-D-xylopyranoside were isolated and characterised. Jung et al., 'HPLC analysis and antioxidant activity of Ulmas davidiana and some flavonoids', Food Chemistry, Elsevier Ltd., NL, vol. 120, no. 1, 1 May 2010, pages 313-318, discloses a study investigating the antioxidative activities of (-)-catechin, (-)-catechin-7-O-β-D-apiofuranoside, and (-)-catechin-7-O-β-D-xylopyranoside in terms of their ability to promote metal chelation, prevent lipid peroxidation, and inhibit DNA cleavage. Park et al., '(-)-Catechin-7-O-β-D-Apiofuranoside Inhibits Hepatic Stellate Cell Activation by Suppressing the STAT3 Signaling Pathway', Cells, vol. 9, no. 1, 20 December 2019, page 30, discloses a study investigating the inhibitory effect on hepatic stellate cell activation by (-)-catechin-7-O-β-D-apiofuranoside.

KR 20060095062 A relates to hair loss prevention and a hair growth promoting composition containing an extract of Ulmus radics cortex. JP 2000095649 A discloses a testosterone-5α-reductase inhibitor comprising one or more extracts from the liquid mixtures of a hydrophilic solvent or water and another component which is selected from the plant group of poplar, Alnus firma, mace, angelica tree, cubeb, neem, Onosma bracteatum and Parumeria, or for the galencical group of Salviae miltiorrhizae radix, nutgall, Juujuka, Mastsufusi, Kojokon (root of Polygonum reynoutria), Bunsinmoku and Alpiniae katsumadiaii Semen, or from the Brazil-derived plant group of Mere and Jakoba.

### [Related art documents]

### [Patent documents]

Korean Patent No. 10-2016002
Korean Patent No. 10-1659516
Korean Patent Application Publication No. 10-2013-0123626
KR 20060095062 A
JP 2000095649 A

### Disclosure of the Invention

### Technical Goals

The present inventor has made efforts for research to develop a hair loss prevention agent derived from natural products that is safer and has no side effects. As a result, the present inventor has experimentally verified that a catechin 7-O-β-D-apiofuranoside compound isolated as a single compound from an extract for supercritical extract residue of a plant of the genus Ulmus davidiana var. japonica inhibits the production of dihydrotestosterone (DHT) derived from testosterone in human dermal papilla cells, and effectively inhibits the apoptosis induced by oxidative stress in human dermal papilla cells. Accordingly, the present invention was completed after confirming that the compound could be developed as a therapeutic agent for alopecia.

Accordingly, an aspect of the present invention is to provide a composition according to the appended claims.

Other aspects and technical features of the present invention will be presented more specifically by the following detailed description of the invention, the claims, and the drawings. Technical Solutions

According to an aspect of the present invention, there is provided a composition for use in preventing, or treating alopecia, in which the composition includes a catechin 7-O-3-D-apiofuranoside compound as an active ingredient.

Catechin 7-O-β-D-apiofuranoside, which is an active ingredient of the composition of an embodiment of the present invention, is a catechin glycoside compound represented by Formula 1 below.

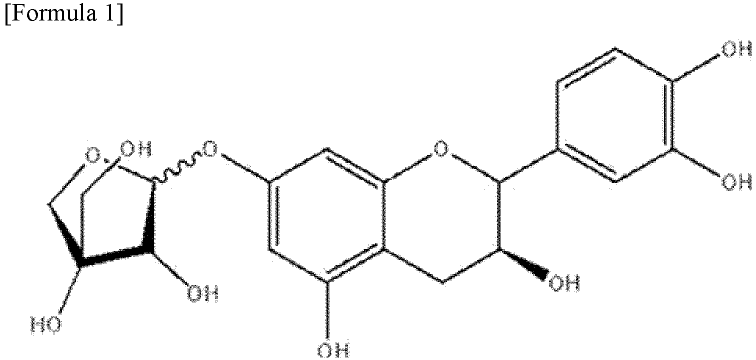

The catechin 7-O-β-D-apiofuranoside, an active ingredient of the composition of an embodiment of the present invention, may use one isolated from natural products, but it is obvious to a person skilled in the art that chemically synthesized ones exhibit the same effect as extracted ones.

The catechin 7-O-β-D-apiofuranoside compound of an embodiment of the present invention may be contained in a large amount in a solvent extract for supercritical extract residue of a plant of the genus Ulmus davidiana var. japonica, which is a natural product extract, and may be obtained from this extract by a conventional single compound isolation method.

In an embodiment of the present invention, the supercritical extract residue of a plant of the genus Ulmus davidiana var. japonica refers to a residue remaining after extracting a plant of the genus Ulmus davidiana var. japonica by a supercritical extraction method.

The plant of the genus Ulmus davidiana var. japonica used in the production of the supercritical extract residue of an embodiment of the present invention includes plants belonging to the order Rosales and the family Ulmaceae, for example, Ulmus davidiana, Ulmus davidiana var. japonica, Ulmus davidiana var. japonica for. Suberosa, Ulmus parvifolia, Ulmus pumila, Ulmus laciniata, Ulmus macrocarpa, and the like. Most representatively, Ulmus davidiana and Ulmus davidiana var. japonica may be used.

Regarding the plant of the genus Ulmus davidiana var. japonica used in the supercritical extraction in an embodiment of the present invention, the whole of the plant of the genus Ulmus davidiana var. japonica; or a part of the plant of the genus Ulmus davidiana var. japonica selected from the group consisting of stem, root, leaf, flower, fruit, and seed may be used. Preferably, the stem, branch, or root of the plant of the genus Ulmus davidiana var. japonica may be used. In an embodiment of the present invention, all or part of the plant of the genus Ulmus davidiana var. japonica may be dried and/or pulverized prior to supercritical extraction.

In an embodiment of the present invention, the supercritical extraction method refers to a method of extracting fragrances, pigments, oils, or functional substances contained in natural products using a fluid in a supercritical state without denaturation.

The fluid in a supercritical state refers to a fluid in a state in which gas and liquid are not distinguished beyond a certain high-temperature and high-pressure limit of a critical point (supercritical point). The supercritical fluid may be, for example, supercritical carbon dioxide. The supercritical carbon dioxide is carbon dioxide in a state exceeding the critical temperature (31°C) and critical pressure (7.5 MPa), and is a fluid having both gas and liquid properties.

The supercritical extraction method in an embodiment of the present invention may be performed by, for example, a pressure change method in which a solute is isolated while the solvency of the supercritical fluid is reduced by expanding a mixture of the supercritical fluid and the solute under reduced pressure at the same temperature as the extraction temperature; or a temperature change method of isolating the supercritical fluid and the solute by increasing the temperature of the supercritical fluid to reduce the solvency, but is not limited thereto.

In an embodiment of the present invention, a co-solvent may be used together with the supercritical fluid, and the co-solvent includes polar solvents commonly used in the art. Preferably, the co-solvent may be selected from the group consisting of alcohol, water, ethylene glycol, polyethylene glycol, propylene carbonate, formic acid, acetic acid, acetonitrile, chlorodifluoromethane, or a mixture thereof.

In an embodiment of the present invention, the vessel for supercritical extraction may be controlled in temperature and pressure, and is not particularly limited as long as it is a vessel configured to contact the extraction raw material and the supercritical fluid.

In an embodiment of the present invention, the pressure in the supercritical extraction may be in the range of 100 to 600 bar, the temperature in the supercritical extraction may be in the range of 10 to 100°C, and the flow rate of the supercritical fluid and co-solvent in the supercritical extraction may be 10 to 100 g/min. A person skilled in the art may appropriately adjust and use the pressure, temperature, and flow rate of the fluid and co-solvent within the above ranges in supercritical extraction.

An extract is obtained through extraction using a solvent for supercritical extract residue of a plant of the genus Ulmus davidiana var. japonica obtained after performing the supercritical extraction method. Preferably, the solvent extraction is performed through a process of contacting supercritical extract residue of Ulmus davidiana var. japonica with an extraction solvent. Prior to the solvent extraction, the supercritical extract residue of Ulmus davidiana var. japonica may be dried and/or pulverized. In an embodiment of the present invention, the extraction solvent may preferably use an alcohol, and the alcohol may be an anhydrous or hydrous lower alcohol having 1 to 4 carbon atoms, such as methanol, ethanol, propanol, butanol, normal-propanol, iso-propanol, and normal-butanol.

In an embodiment of the present invention, fractions may be obtained by performing additional fractionation on the solvent extract of the supercritical extract residue of Ulmus davidiana var. japonica.

The fractionation is carried out by bringing the fractionation solvent into contact with the extract, similarly to extraction. The solvent used in the fractionation includes (a) water, (b) alcohol, (c) a mixed solvent of the lower alcohol and water, (d) acetone, (e) ethyl acetate, (f) chloroform, (g) butyl acetate, (h) 1,3-butylene glycol, (i) hexane, and (j) diethyl ether. Preferably, it may be a fraction obtained using ethyl acetate.

From the fraction, catechin 7-O-β-D-apiofuranoside may be isolated using a common single compound isolation method known in the art such as chromatography. As a method for isolating and purifying a single compound that may be used in an embodiment of the present invention, for example, TLC (Thin Layer Chromatography) or HPLC (High Performance Liquid Chromatography) may be used.

As used herein, the term "including as an active ingredient" means that the catechin 7-O-β-D-apiofuranoside compound of an embodiment of the present invention is included in an amount sufficient to achieve the efficacy of preventing, or treating alopecia.

As demonstrated in a specific example of the present specification, the active compound of an embodiment of the present invention, catechin 7-O-β-D-apiofuranoside, suppresses the conversion of testosterone into dihydrotestosterone (DHT) by 5-alpha-reductase in human-derived dermal papilla cells (HFDPC). Accordingly, the compound of an embodiment of the present invention may exhibit effects of preventing, and treating male pattern hair loss.

As demonstrated in another embodiment of the present specification, the active compound of an embodiment of the present invention, catechin 7-O-β-D-apiofuranoside, suppresses apoptosis of dermal papilla cells (HFDPC) by oxidative stress. Accordingly, the compound of an embodiment of the present invention may be used for the purpose of preventing or treating alopecia, particularly stress-induced alopecia.

As used herein, the term "hair loss" refers to a condition in which there is no hair in an area where hair should normally exist due to abnormal hair loss, and the term "alopecia" refers to a condition caused by such hair loss.

As used herein, the term "male pattern alopecia" is also referred to as androgenetic alopecia, and means alopecia caused by genetic causes and effects of male hormones or aging.

More specifically, in an embodiment of the present invention, it may be alopecia caused by dihydrotestosterone (DHT), which is produced by converting testosterone, a male hormone, by the action of 5-alpha-reductase.

As used herein, the term "stress-induced alopecia" refers to alopecia caused by psychological or physical stress, and includes stress-induced alopecia areata and stress-induced telogen alopecia.

As used herein, the term "stress-induced alopecia" includes not only human stress-induced alopecia but also stress-induced alopecia occurring in animals other than humans.

The composition of an embodiment of the present invention may be provided in the form of a pharmaceutical composition for preventing or treating alopecia, and the pharmaceutical composition may include a pharmaceutically acceptable carrier.

As used herein, the term "prevention" refers to inhibition of symptoms due to hair loss or symptoms due to complications of hair loss, and the term "treatment" refers to reduction or elimination of symptoms due to already occurring hair loss or symptoms due to complications of hair loss.

The pharmaceutically acceptable carrier included in the composition of an embodiment of the present invention is one commonly used in formulations and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. The pharmaceutical composition of an embodiment of the present invention may further include, in addition to aforesaid ingredients, a lubricant, a wetting agent, a sweetener, a flavor, an emulsifier, a suspending agent, a preservative, or the like. Suitable pharmaceutically acceptable carriers and formulations are described in detail in Remington's Pharmaceutical Sciences (19th ed., 1995).

The pharmaceutical composition of an embodiment of the present invention may be administered orally or parenterally, and in the case of parenteral administration, it may be administered by intravenous injection, subcutaneous injection, intramuscular injection, intraperitoneal injection, transdermal administration, or the like.

A suitable dosage of the pharmaceutical composition of an embodiment of the present invention may vary depending on various factors including formulation method, administration method, age, weight, gender or pathological status of a patient, diet, administration time, administration route, excretion rate and response sensitivity. Usually, a skilled practitioner may readily determine and prescribe the dosage effective for the desired treatment or prophylaxis.

The pharmaceutical composition of an embodiment of the present invention may be administered in an amount of 0.001 to 10,000 mg/kg a day.

The pharmaceutical composition of an embodiment of the present invention may be formulated according to a method that may be easily carried out by those skilled in the art using a pharmaceutically acceptable carrier and/or excipient to be prepared in unit-dose forms or by pouring into multi-dose packages. In this connection, the formulation may be in the form of a solution in oil or an aqueous medium, a suspension, aor an emulsion, or in the form of elixirs, powders, granules, a tablet, or a capsule, and may further include a dispersing agent or a stabilizing agent.

### Advantageous Effects

The catechin 7-O-β-D-apiofuranoside compound of an embodiment of the present invention inhibits the production of dihydrotestosterone (DHT) converted from testosterone by 5-alpha-reductase in human-derived dermal papilla cells, and inhibits the apoptosis induced by oxidative stress in human dermal papilla cells, and accordingly, can be used as an active substance for the treatment, or prevention of alopecia.

### Brief Description of Drawings

FIG. 1A is a process flowchart summarizing an isolation process of the compound of an embodiment of the present invention.
FIG. 1B shows the chromatographic results for identifying catechin 7-O-β-D-apiofuranoside in an ethanol extract for supercritical extract residue of branches of Ulmus davidiana var. japonica.
FIG. 1C shows the chromatographic results for identifying the catechin 7-O-β-D-apiofuranoside in a fraction fractionated with ethyl acetate for the ethanol extract for supercritical extract residue of branches of Ulmus davidiana var. japonica.
FIG. 1D is a result of identifying a catechin 7-O-β-D-apiofuranoside compound obtained by isolating the fraction fractionated with ethyl acetate from the ethanol extract for supercritical extract residue of branches of Ulmus davidiana var. japonica by Prep-LC.
FIG. 2A is a TOF-MS analysis result of the catechin 7-O-β-D-apiofuranoside compound.
FIG. 2B is 13C-NMR data of the catechin 7-O-β-D-apiofuranoside compound.
FIG. 2C is 1H-NMR data of the catechin 7-O-β-D-apiofuranoside compound.
FIG. 3 is a result of measuring the effect of the catechin 7-O-β-D-apiofuranoside compound on the production of dihydrotestosterone (DHT) in human-derived dermal papilla cells. *P<0.001 vs blank group. #P<0.001 and †P<0.05 vs testosterone group.
FIG. 4 is an experimental result showing that the catechin 7-O-β-D-apiofuranoside compound inhibits Bax protein expression, increases Bcl-2 protein expression, and inhibits PARP-1 protein expression in dermal papilla cells treated with hydrogen peroxide.
FIG 5A is a graph showing the effect of the catechin 7-O-β-D-apiofuranoside to promote the expression of IGF conducive to hair growth in a concentration-dependent manner in H₂O₂-treated dermal papilla cells. *P<0.001 vs normal group. **P<0.005 vs H2O2 control group.
FIG. 5B is a graph showing the effect of the catechin 7-O-β-D-apiofuranoside to inhibit the expression of TGF-β1 in a concentration-dependent manner in H2O2-treated dermal papilla cells. *P<0.001 vs normal group. **P<0.005 vs H2O2 control group.

### Best Mode for Carrying Out the Invention

Specific examples described in the present specification are intended to represent embodiments or examples of the present invention, and the scope of the present invention is not limited thereby. It will be apparent to those skilled in the art that variations and other uses of the present invention do not depart from the scope of the present invention as defined in the appended claims of the present specification.

### Examples

### 1. Experimental Materials and Methods

### (1) Isolation of Catechin 7-O-β-D-Apiofuranoside Compound

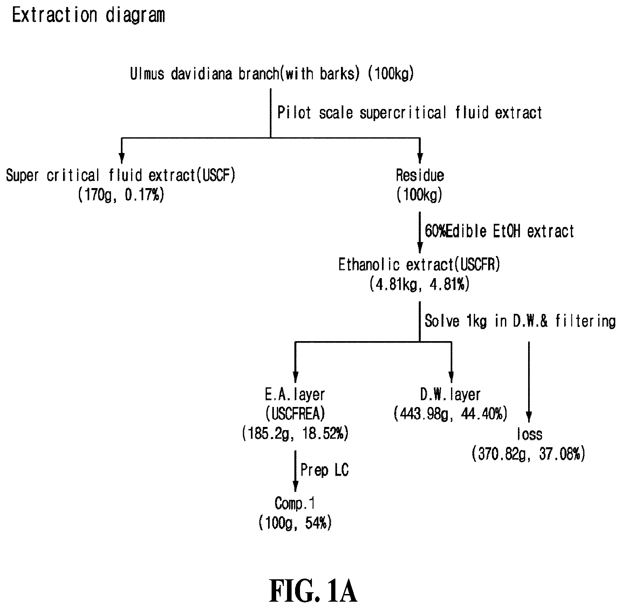

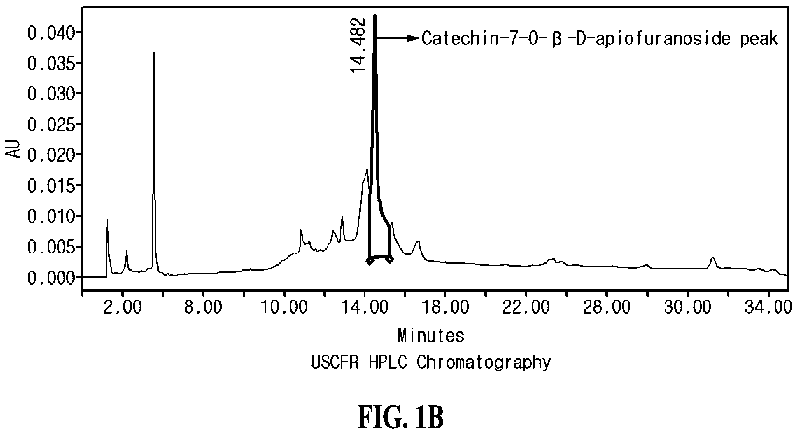

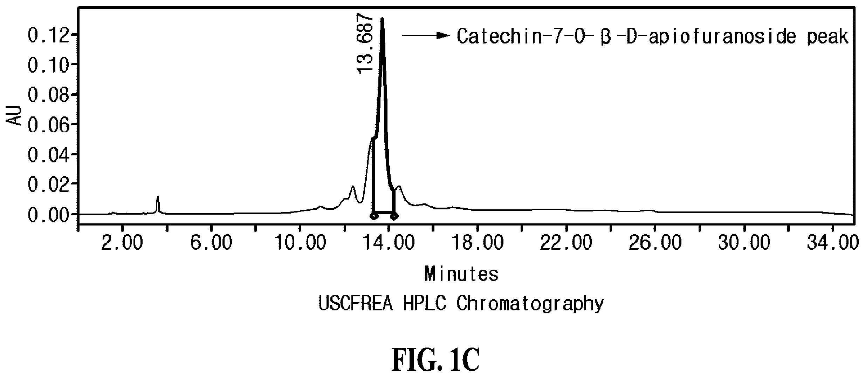

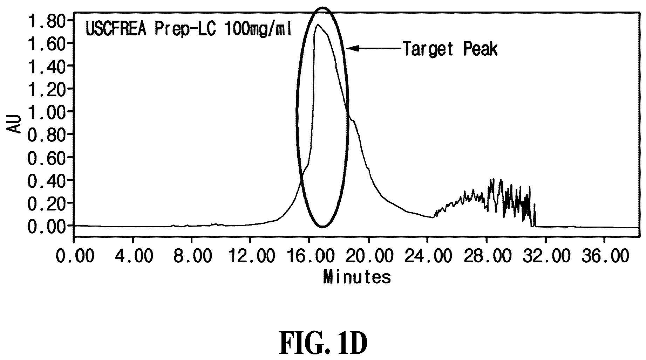

Supercritical extraction of branches of Ulmus davidiana var. japonica in this experiment was performed using equipment for producing supercritical fluid extraction (SCFE-P400, Ilsin Autoclave, Daejeon, Korea). The branches of Ulmus davidiana var. japonica were purchased from Yangnyeong Market in Seoul, foreign substances were removed, washed, and then dried in the shade to be used as experimental materials. 100 kg of the dried sample was pulverized so as to pass through a 200-mesh pulverizing net, and the temperature of the pulverized branches of Ulmus davidiana var. japonica was maintained by adjusting the temperature of the extraction tank to 50°C. When the temperature was stabilized, the branches of Ulmus davidiana var. japonica as a sample were put in the equipment, CO₂ gas was maintained at an equal pressure, and then the injection was performed by adjusting the control valve until the experimental pressure condition of 400 bar was reached through the line using a high-pressure pump. After reaching the set pressure, extraction was carried out by putting a total of 60 to 80 L of ethanol (alcohol: edible alcohol) at a rate of 0.5 to 0.6 L per minute to the bottom of the extraction tank for 160 minutes, and extraction was completed by flowing CO2 using a high-pressure pump for 60 to 120 minutes at a set pressure and temperature to remove residual ethanol remaining in the sample. After the supercritical extraction of the branches of Ulmus davidiana var. japonica as above, 100 kg of the obtained sample for supercritical extract residue of Ulmus davidiana var. japonica was dried in the shade, extracted once with 60% ethanol at room temperature, and filtered. The extract was concentrated under reduced pressure and lyophilized to obtain a final weight of 4.81 kg. 1 kg of the ethanol extract ("USCFR") of the obtained supercritical extract residue of Ulmus davidiana var. japonica was quantified and subjected to ethyl acetate solvent fractionation using a separatory funnel to obtain 185.2 g of ethyl acetate solvent fraction ("USCFREA"). Using Prep-LC (Waters, USA) from the obtained ethyl acetate solvent fraction ("USCFREA"), when using silica gel resin, the mobile phase solvent was isolated using Chloroform : Methanol : Water (70 : 30 : 4). When using the C18 column resin, a single compound was purified using MeOH : Water (0 - 100%) mobile phase conditions (*see* FIGS. 1A to 1D).

### (2) Analysis of Indicator Compound in Extract Using TOF-MS

The molecular weight and molecular structure of catechin 7-O-β-D-apiofuranoside, the main ingredient in the supercritical residue ethanol extract of branches of Ulmus davidiana var. japonica, were identified using Direct Probe with a high resolution time-of-flight mass spectrometer using high vacuum and high sensitivity detector through TMP.

Sample information and analysis conditions are described in Table 1 below.

**[Table 1]**

| Sample information | | |
|---|---|---|
| Item | Molecular weight | Formula |
| Catechin-7-O-β-D-apiofuranoside | 422.38 g/mol | 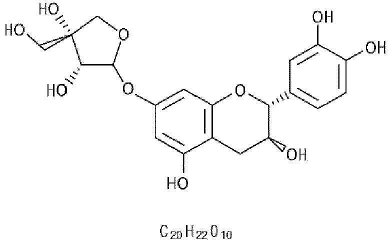 |

| Analysis condition | | |
|---|---|---|
| Item | Condition | |
| Mass Range | ~ 1,000 | |
| Acquisition rate | Up to 50 spectra/s | |
| Mass measurement accuracy | 1.5 mDa or 4 ppm | |

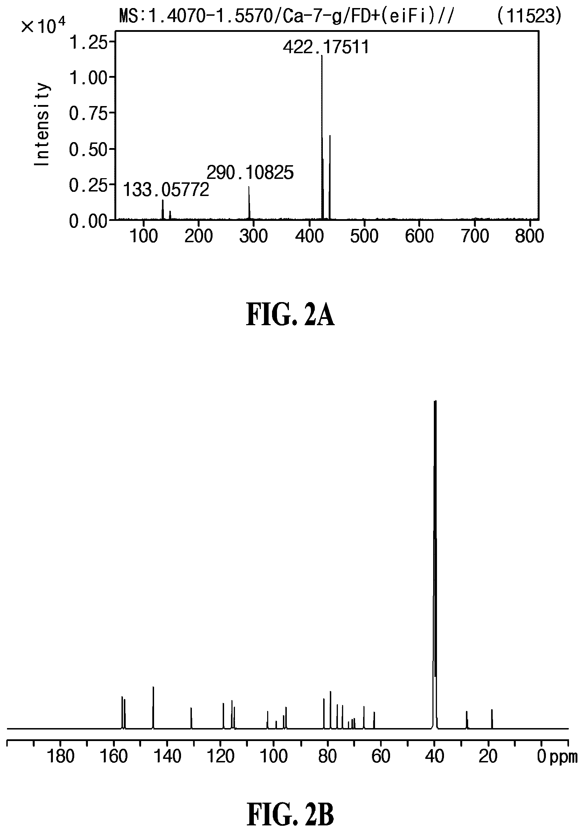

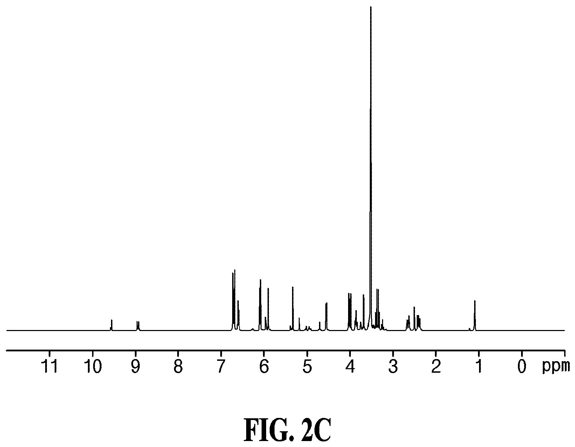

The analysis result of TOF-MS is shown in FIG. 2A, 13C-NMR data is shown in FIG. 2B, and 1H-NMR data is shown in FIG. 2C.

The results of structural analysis of the catechin 7-O-β-D-apiofuranoside compound of an embodiment of the present invention are shown below.

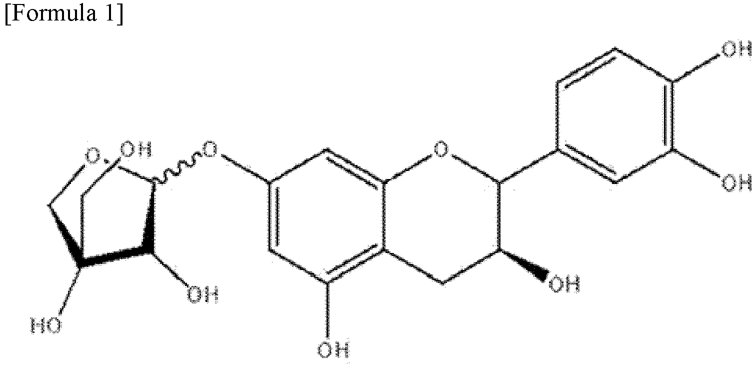

High Resolution TOF MS m/z: 422.17511 [M]+;1H-NMR (400 MHz, DMSO-d6+D2O):6.74(H-2',1H,d,J=2.0Hz),6.69(H-5',1H,d,J=8.4Hz),6.59(H-6',1H,ddJ=2.0,8.4Hz),6.09(H-8,1H,d,J=2.4Hz),5.90(H-6,1H,J=2.4Hz),5.33(H-1",1H,d,J=4.0Hz),4.55(H-2,1H,d,J=7.2Hz),4.03(H-2",1H,d,J=4.0Hz),4.00(H-4a",1H,d,J=9.6Hz),3.89(H-3,1H,m), 3.68(H-4b",1H,d,J=9.6Hz),3.45(H-5",2H,m),2.65(H-4a,1H,dd,J=4.8,16.0Hz),2.40(H-4b,1H,dd,J=8.0,16.0z); 13C-NMR(100MHz,DMSO-d6+D2O):δ156.7(C-7),156.5(C-5),155.7(C-9),145.2(C-4'),145.1(C-3'),130.8(C-1'),118.8(C-6'),115.6(C-5'),114.7(C-2'),107.3(C-1"),102.1(C-10),96.0(C-8),95.3(C-6),81.4(C-2),78.9(C-3"),76.3(C-2"),74.3(C-4"),66.3(C-3),62.4(C-5"),27.8(C-4).

### (3) Cell Culture

Human-derived hair follicle dermal papilla cells (HFDPC) used in this Example were purchased from PromoCell (Heidelberg, Germany) and used as cells exhibiting the characteristics of hair growth and maintenance. HFDPC cells were inoculated in a papilla cell-specific medium (PromoCell, Heidelberg, Germany or Cell biologics Inc, IL, USA) recommended by the supplier, subcultured for 24 to 48 hours at 37°C and 5% CO2 conditions, and used for experiments.

### (4) Dihydrotestosterone Production Inhibitory Activity Assay

It was experimented whether the catechin 7-O-β-D-apiofuranoside compound inhibited a process of the production of dihydrotestosterone from testosterone in dermal papilla cells.

Quantitative analysis of dihydrotestosterone (DHT) was performed by immunochemical quantitative analysis using an ELISA kit. A DHT assay ELISA kit was purchased from Alpco (NH, USA) and used. The experiment using the kit was performed according to the instructions provided by the manufacturer, and the result was derived by converting the amount of antigen (pg/µg) per volume (ml) of cell lysate into a concentration unit.

### (5) Inhibitory Effect on Apoptosis in Dermal Papilla Cells

It was experimented whether the catechin 7-O-β-D-apiofuranoside compound might inhibit apoptosis caused by oxidative stress in dermal papilla cells. In this experiment, the oxidative stress inhibitory effect on dermal papilla cells (HFDPC) induced by hydrogen peroxide (H2O2) was measured through the MTT experiment. Dermal papilla cells were dispensed at 5 × 10⁶ cells/mL in a 96-well plate, and after 12 hours, 600 µM of H2O2 and sample were simultaneously treated for 4 hours. The dermal papilla cells in this state were treated with MTT stock solution to obtain a final concentration of 0.2 mg/ml, left at 37°C for 4 hours, and then purple-stained formazan was dissolved in DMSO and absorbance was measured at 570 nm. Cell viability was expressed as a % value relative to the control group.

### (6) Western Blot Analysis

After treating the dermal papilla cells (HFDPC) with H2O2 and the sample, the cells were collected at each time zone and the cells were lysed in a cell lysis buffer (RIPA buffer). After the sample was centrifuged at 13,000 g for about 10 to 15 minutes, only the supernatant was taken. The protein concentration of the supernatant was measured by a BCA protein assay kit (Bio-Rad, CA, USA) and then heated at 95°C for 10 minutes. The prepared sample was electrophoresed on SDS-polyacrylamide gel, transferred to a polyvinylidenedifluoride membrane (PVDF, Bio-rad, CA, USA), blocked with 5% BSA, and reacted with the indicated specific antibody. Then, HRP-conjugated secondary antibody (Santacruz, CA, USA) was reacted with the sample and detected by ECL system (Bio-Rad, MO, USA). As primary antibodies against Bax-1, Bcl-2, PARP-1, and β-actin, Santacruz antibodies (CA, USA) were used.

### (7) ELISA Assay

An ELISA kit was purchased and used to perform immunochemical quantitative analysis related to hair growth promotion and hair loss inhibition. TGF-β and IGF-1 kits were purchased from R&D systems (CA, USA) and used. The test was conducted using each kit according to the supplier's manual, and the result was derived by converting the amount (pg or µg) of antigen per volume (ml) of cell lysate into a concentration unit.

### (8) Statistical Processing

All experiments were repeated three times, and the average value and standard deviation were presented. Also, SPSS (Statistical Package for Social Science, ver. 18, IBM, Chicago IL. USA) was used to verify significance by Student's t-test on the average value of the experimental group and the control group at the level of P<0.05.

### 2. Experiment Result

### (1) Measurement Result of Dihydrotestosterone (DHT) Inhibitory Activity of Catechin 7-O-β-D-Apiofuranoside

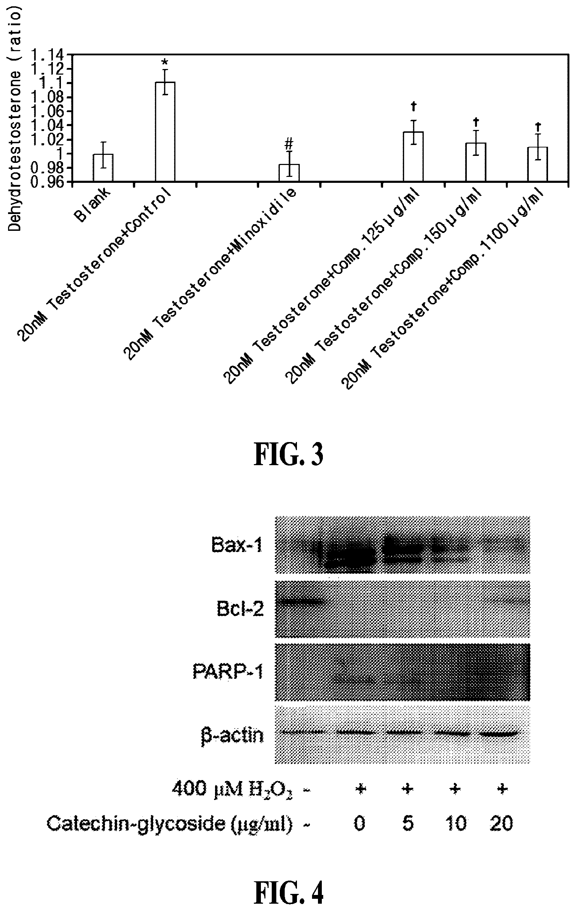

As a result of treating dermal papilla cells (HFDPC) with 20 nM testosterone, it was identified that the production of dihydrotestosterone (DHT) was statistically significantly increased. Next, in order to identify the ability of catechin 7-O-β-D-apiofuranoside to inhibit DHT production, a comparative experiment was conducted using minoxidil, which is well known as a drug for preventing male pattern hair loss, as a positive control group. In the positive control group treated with minoxidil (10 µM), it was identified that the production of DHT was significantly inhibited. In the case of treatment with catechin 7-O-β-D-apiofuranoside (125 µg/ml, 150 µg/ml, 1100 µg/ml), although not as strong as minoxidil, it was identified that the effect of inhibiting DHT production was statistically significant in a concentration-dependent manner. Even when compared with the negative control group, DHT production was inhibited statistically significantly (FIG. 3).

### (2) Bax Protein Expression Inhibitory Effect

Hydrogen peroxide (H2O2) treated to induce apoptotic cytotoxicity of dermal papilla cells (HFDPC) was identified to show 50% apoptosis at a concentration of 600 µM, and thus the concentration was used for the test. Bax (Bcl-2-associated X protein) as a representative apoptosis-inducing protein in apoptotic cytotoxicity was identified to significantly increase the expression of Bax molecules by treatment with H2O2. In contrast, as a result of pretreatment with the catechin 7-O-β-D-apiofuranoside compound at each concentration, it was demonstrated that the expression of Bax was significantly reduced in a concentration-dependent manner (FIG. 4). These results suggest that catechin 7-O-β-D-apiofuranoside effectively inhibits stress-induced apoptosis of dermal papilla cells.

### (3) Effect of Increasing Bcl-2 Protein Expression

In order to induce apoptotic cytotoxicity of dermal papilla cells (HFDPC) in the same way as the Bax expression inhibitory ability evaluation method, dermal papilla cells were treated with 600 µM of hydrogen peroxide (H2O2), and the induced apoptosis was evaluated. Bcl-2 is a representative protein molecule involved in apoptosis inhibition, and it was observed that the expression of Bcl-2 molecule was significantly reduced when HFDPC was treated with H2O2. In contrast, as a result of treatment with the catechin 7-O-β-D-apiofuranoside compound at each concentration, it was demonstrated that USCFR significantly increased the expression of Bcl-2 in a concentration-dependent manner in the presence of H2O2 (FIG. 4). These results suggest that catechin 7-O-β-D-apiofuranoside effectively inhibits stress-induced apoptosis of dermal papilla cells.

### (4) PARP-1 Protein Expression Inhibitory Effect

PARP-1 uses intracellular NAD as a substrate to induce poly-ADP ribosylation of target proteins in the cell nucleus, thereby activating signaling involved in lower apoptosis. For this reason, the activation of PARP-1 is used as a leading indicator of apoptosis, and is particularly used as a representative marker related to apoptosis. Treatment with 600 µM of hydrogen peroxide (H2O2) significantly increased intracellular PARP-1 expression in HFDPC. These results demonstrate that apoptosis is in progress in HFDPC. In contrast, as a result of treatment with the catechin 7-O-β-D-apiofuranoside compound at each concentration, it was demonstrated that the compound significantly inhibited the protein expression level of PARP-1 in a concentration-dependent manner in the presence of H2O2 (FIG. 4). These results suggest that the catechin 7-O-β-D-apiofuranoside compound effectively inhibits stress-induced apoptosis of dermal papilla cells.

### (5) Expression Promotion Effect of IGF-1 and Expression Inhibition Effect of TGF-β1

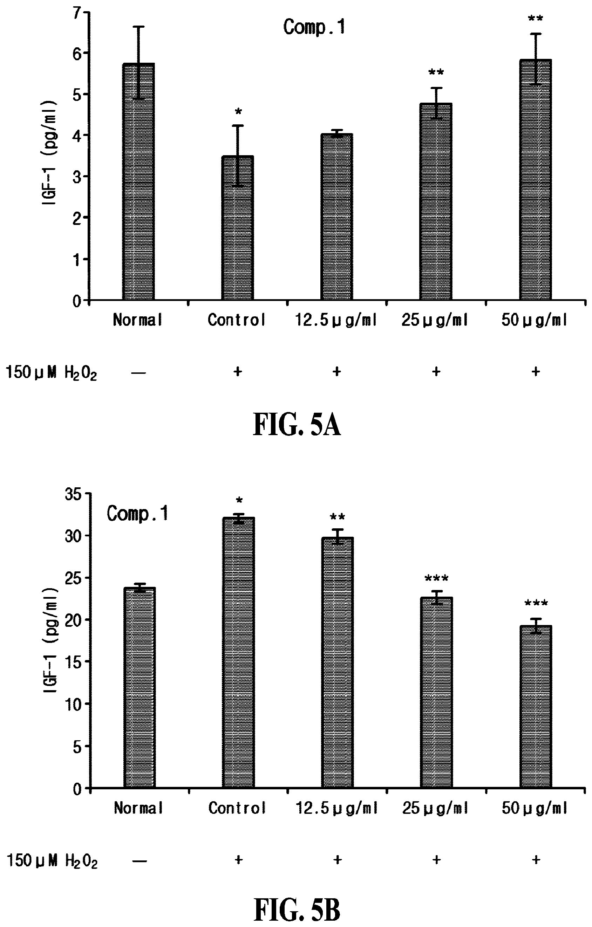

In this experiment, the effects of catechin 7-O-β-D-apiofuranoside compound to promote the expression of IGF-1 that is helpful for hair growth (FIG. 5A) and to inhibit TGF-β1 that inhibits hair growth and accelerates hair loss (FIG. 5B) were identified. Experimental results of items (2) to (4) above show that catechin 7-O-β-D-apiofuranoside effectively inhibits apoptosis of dermal papilla cells caused by oxidative stress, and is effective in stress-induced hair loss.

## Claims

1. A composition for use in preventing or treating alopecia, the composition comprising a catechin 7-O-β-D-apiofuranoside compound as an active ingredient.

2. The composition for use according to claim 1, wherein the catechin 7-O-β-D-apiofuranoside compound has an activity of inhibiting production of dihydrotestosterone (DHT) from testosterone.

3. The composition for use according to claim 1, wherein the alopecia is induced by dihydrotestosterone (DHT).

4. The composition for use according to claim 1, wherein the catechin 7-O-β-D-apiofuranoside compound has an activity of inhibiting apoptosis caused by oxidative stress in dermal papilla cells.

5. The composition for use according to claim 1, wherein the alopecia is male pattern alopecia or stress-induced alopecia.

6. The composition for use according to claim 1, wherein the composition is a pharmaceutical composition.

## Patentansprüche

1. Zusammensetzung zur Verwendung bei
der Vorbeugung und Behandlung von Alopezie, wobei die Zusammensetzung eine Catechin-7-O-β-D-Apiofuranosid-Verbindung als Wirkstoff umfasst.

2. Zusammensetzung zur Verwendung nach Anspruch 1, wobei die Catechin-7-O-β-D-Apiofuranosid-Verbindung eine hemmende Wirkung auf die Produktion von Dihydrotestosteron (DHT) aus Testosteron hat.

3. Zusammensetzung zur Verwendung nach Anspruch 1, wobei die Alopezie durch Dihydrotestosteron (DHT) induziert ist.

4. Zusammensetzung zur Verwendung nach Anspruch 1, wobei die Catechin-7-O-β-D-Apiofuranosid-Verbindung eine hemmende Wirkung auf die Apoptose, die durch oxidativen Stress in dermalen Papillenzellen verursacht wird, hat.

5. Zusammensetzung zur Verwendung nach Anspruch 1, wobei die Alopezie eine Alopezie nach männlichem Muster oder eine Stress-induzierte Alopezie ist.

6. Zusammensetzung zur Verwendung nach Anspruch 1, wobei die Zusammensetzung eine pharmazeutische Zusammensetzung ist.

## Revendications

1. Composition pour utilisation dans la prévention ou le traitement de l'alopécie, la composition comprenant un composé de catéchine-7-O-β-D-apiofuranoside en tant que substance active.

2. Composition pour utilisation selon la revendication 1, dans laquelle le composé de catéchine-7-O-β-D-apiofuranoside a une activité d'inhibition de la production de dihydrotestostérone (DHT) à partir de la testostérone.

3. Composition pour utilisation selon la revendication 1, où l'alopécie est induite par la dihydrotestostérone (DHT).

4. Composition pour utilisation selon la revendication 1, dans laquelle le composé de catéchine-7-O-β-D-apiofuranoside a une activité d'inhibition de l'apoptose causée par le stress oxydant dans les cellules des papilles dermiques.

5. Composition pour utilisation selon la revendication 1, où l'alopécie est une alopécie androgénétique masculine ou une alopécie induite par le stress.

6. Composition pour utilisation selon la revendication 1, où la composition est une composition pharmaceutique.
